(19) Europäisches Patentamt / European Patent Office / Office européen des brevets

(11) **EP 3 786 617 B1**

(12) **EUROPEAN PATENT SPECIFICATION**

(45) Date of publication and mention
of the grant of the patent:
**07.08.2024 Bulletin 2024/32**

(21) Application number: **18915825.6**

(22) Date of filing: **19.12.2018**

(51) International Patent Classification (IPC):
$G01N\ 27/06$ (2006.01)   $G01N\ 21/31$ (2006.01)
$G01N\ 33/18$ (2006.01)   $A01G\ 31/00$ (2018.01)
$G16C\ 20/30$ (2019.01)   $G16C\ 20/20$ (2019.01)

(52) Cooperative Patent Classification (CPC):
**G01N 27/06; A01G 31/00; C05G 5/23; G01N 33/18;**
G01N 31/22; G01N 31/227; G16C 20/20;
G16C 20/30; Y02P 60/21

(86) International application number:
**PCT/CN2018/122033**

(87) International publication number:
**WO 2019/205673 (31.10.2019 Gazette 2019/44)**

(54) **METHOD FOR CALCULATING ION EC CONTRIBUTION RATE AND CONDUCTIVITY OF NUTRIENT SOLUTION ON BASIS OF ION ACTIVITY**

VERFAHREN ZUR BERECHNUNG DER IONEN-EC-BEITRAGSRATE UND LEITFÄHIGKEIT EINER NÄHRLÖSUNG AUF BASIS DER IONENAKTIVITÄT

PROCÉDÉ DE CALCUL DU TAUX DE CONTRIBUTION D'EC IONIQUE ET DE LA CONDUCTIVITÉ D'UNE SOLUTION NUTRITIVE EN FONCTION D'UNE ACTIVITÉ IONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.04.2018 CN 201810380292**

(43) Date of publication of application:
**03.03.2021 Bulletin 2021/09**

(73) Proprietor: **China Agricultural University**
**Beijing 100083 (CN)**

(72) Inventors:
• **HE, Dongxian**
  **Beijing 100083 (CN)**
• **SONG, Jinxiu**
  **Beijing 100083 (CN)**
• **XU, Lin**
  **Beijing 100083 (CN)**
• **DU, Weifen**
  **Beijing 100083 (CN)**
• **KOZAI, Toyoki**
  **Beijing 100083 (CN)**

(74) Representative: **ABG Intellectual Property Law, S.L.**
**Avenida de Burgos, 16D**
**Edificio Euromor**
**28036 Madrid (ES)**

(56) References cited:
**CN-A- 102 645 477**   **CN-A- 103 249 478**
**CN-A- 108 896 498**

• **MOREIRA BARRADAS J M ET AL: "A model to formulate nutritive solutions for fertigation with customized electrical conductivity and nutrient ratios", IRRIGATION SCIENCE, SPRINGER BERLIN HEIDELBERG, BERLIN/HEIDELBERG, vol. 36, no. 3, 3 March 2018 (2018-03-03), pages 133 - 142, XP036485568, ISSN: 0342-7188, [retrieved on 20180303], DOI: 10.1007/S00271-018-0569-9**

EP 3 786 617 B1

- MOREIRA BARRADAS J M ET AL: "A Decision Support System-Fertigation Simulator (DSS-FS) for design and optimization of sprinkler and drip irrigation systems", COMPUTERS AND ELECTRONICS IN AGRICULTURE, ELSEVIER, AMSTERDAM, NL, vol. 86, 23 February 2012 (2012-02-23), pages 111 - 119, XP028399127, ISSN: 0168-1699, [retrieved on 20120315], DOI: 10.1016/J.COMPAG.2012.02.015
- VISCONTI F. ET AL: "An empirical equation to calculate soil solution electrical conductivity at 25°C from major ion concentrations", EUROPAN JOURNAL OF SOIL SCIENCE, vol. Consideration of ionic activity instead of anali61, no. 6, 17 November 2010 (2010-11-17), GB, pages 980 - 993, XP055866411, ISSN: 1351-0754, DOI: 10.1111/j.1365-2389.2010.01284.x
- "Nutrient Solutions for Hydroponic Systems", 1 January 2012, INTECHOPEN, ISBN: 978-953-51-0386-8, article TREJO-TÉLLEZ LIBIA I ET AL: "Nutrient Solutions for Hydroponic Systems", XP055871908, DOI: 10.5772/37578
- HANPING MAO ET AL: "Regression Model of the mother liquid dosage and the value of EC/pH in Facility Cultivation", JOURNAL OF AGRICULTURAL MECHANIZATION RESEARCH, vol. 2, 29 February 2012 (2012-02-29), pages 149 - 151, XP055687627
- SONG JINXIU ET AL: "Estimating EC and ionic EC contribution percentage of nutrient solution based on ionic activity", INTERNATIONAL JOURNAL OF AGRICULTURAL AND BIOLOGICAL ENGINEERING, vol. 12, no. 2, 1 January 2019 (2019-01-01), CN, pages 42 - 48, XP055866075, ISSN: 1934-6344, DOI: 10.25165/j.ijabe.20191202.4399
- MAO, HANPING ET AL.: "Regression Model of the mother liquid dosage and the value of EC/pH in Facility Cultivation", JOURNAL OF AGRICULTURAL MECHANIZATION RESEARCH, vol. 2, 29 February 2012 (2012-02-29), pages 149 - 151, XP055687627
- QIU, XUEFENG ET AL.: "The On-line Measurement and Estimation of the Nutritive Medium Ingredient", JOURNAL OF UNIVERSITY OF SCIENCE AND TECHNOLOGY OF CHINA, vol. 30, no. 3, 30 June 2000 (2000-06-30), pages 351 - 355, XP055649464
- SAVVAS, DIMITRIOS: "Automated Management of Nutrient Solutions Based on Target Electrical Conductivity, pH, and Nutrient Concentration Ratios", JOURNAL OF PLANT NUTRITION, vol. 22, no. 9, 31 December 1999 (1999-12-31), pages 1415 - 1432, XP055649471
- CHEN, LISHA ET AL.: "Ionic Liquid Solution of the Average Activity Coefficient of Determination", JOURNAL OF LIAOCHENG UNIVERSITY, NATURAL SCIENCE EDITION, vol. 23, no. 1, 1 March 2010 (2010-03-01), pages 30 - 34, XP055687631

## Description

## BACKGROUND

### Technical Field

The present invention belongs to the fields of soilless culture technologies and technologies of integrated management of water-fertilizer. Specifically, the present invention relates to a method for calculating an ionic EC contribution percentage and EC of nutrient solution based on ionic activity as defined in claim 1.

### Related Art

Soilless culture is widely recognized by greenhouse vegetable industry because of its advantages of saving water, fertilizer, labor, and promoting production. The nutrient solution irrigation system based on regulation and control of electrical conductivity (EC) and potential of hydrogen (pH) can roughly meet the demands of plant growth and development. However, it is difficult to ensure the changing demands of crops for specific inorganic ions at different growth stages. Therefore, whether the feedback control on the ionic concentration in a nutrient solution can be performed according to the demands of crop growth is essential to achieve high yield and high quality production in greenhouse vegetable. However, there are problems in inorganic ion dynamic detection technology by using ion selective electrodes, such as low accuracy, short service life, and high price. As a result, the nutrient solution irrigation system based on regulation and control of ionic concentration has not been applied to the actual production, and the main equipment is still based on dynamic regulation and control of EC and pH of a nutrient solution. Therefore, for applications of the nutrient solution having specific formula to crop under protected cultivation, if a new method for controlling each ionic concentration in the nutrient solution by feedback of EC and pH is explored, the technical restriction for ion selective electrodes can be broken through, thus making a nutrient solution irrigation system which uses EC and pH sensors to dilute the concentrated mother solution achieve the feedback regulation and control for each ionic concentration of main compositions in the nutrient solution.

EC is a result of comprehensive effect of the effective concentration of each ion constituting a nutrient solution on the mixed solution. The contribution of each ion to EC of the nutrient solution is not only related to the molar concentration of the ion, but also closely related to the charge number carried by the ion and the volume parameter of the ion. Regulating and controlling EC of nutrient solution can only reflect the change of total salt content or comprehensive ionic concentration in a nutrient solution, but cannot accurately figure out each ionic concentration level of main compositions in the nutrient solution. The pH of a nutrient solution also indirectly affects the effectiveness of many inorganic ions. Too high pH will lead to precipitation of iron, manganese, copper and zinc ions, and especially, the activity of iron ion is significantly affected. Lower pH can cause the absorption restriction of calcium by crops due to the antagonism of hydrogen ions. In order to regulate and control each ionic concentration of main compositions in a nutrient solution to adapt dynamic demands of crops for the main inorganic ions at different growth and development stages, the ionic EC contribution percentage of each ion of main compositions in the nutrient solution concentration to the mixed solution can be calculated based on its activity. Thus, quantitative relationship between each ionic concentration of main compositions in the nutrient solution with a specific formula and its EC can be determined, which provides a theoretical basis and technical support for the dynamic feedback regulation and control of each ionic concentration in the nutrient solution.

Moreira Barradas et al., A model to formulate nutritive solutions for fertigation with customized electrical conductivity and nutrient ratios. Irrig Sci 36, 133-142 (2018) discloses a model relating partial mass concentration (NSC) of several nutritive salts (NS) in solution to its resulting EC and ratio of nutritive elements (NE).

Moreira Barradas et al., A Decision Support System-Fertigation Simulator (DSS-FS) for design and optimization of sprinkler and drip irrigation systems, Computers and Electronics in Agriculture, Volume 86, 2012, Pages 111-119 discloses a decision support system (DSS) and a Fertigation module (FS - Fertigation Simulator). The DSS-FS system is intended to support design and optimization of irrigation and fertigation systems while increasing their environmental sustainability. Based upon soil fertility information and crop uptake tables together with pH and EC limits, the DSS-FS is able to regulate the system's injection rate according to the concentration of several nutritive salts in each of the system's mother solution tanks.

## SUMMARY

The objective of the present invention is to, for a problem that a concentration level of each inorganic ion is difficult to be reflected by regulation and control of EC and pH of existing nutrient solution irrigation system, provide a method for calculating an ionic EC contribution percentage and EC of a nutrient solution based on ionic activity, and explore a new method for inversely calculating each ionic concentration of main compositions in a nutrient solution using the ionic EC contribution percentage and actual measurement of EC, thereby providing a theoretical basis and a technical support for realizing feedback regulation and control of each ionic concentration in a nutrient solution.

To achieve the objective of the present invention, definition and calculation method for ionic EC contribution percentage of a nutrient solution are proposed, and statistical regression equations for estimating EC of a nutrient solution based on ionic activity is proposed. Thus, a quantitative relationship between each ionic concentration of the main compositions and EC of the nutrient solution can be determined, and each ionic concentration is inversely calculated by using the ionic EC contribution percentage and the actually measured EC of the nutrient solution. The method of the present invention is defined in claim 1. The dependent claims describe preferred embodiments of the method.

Specific technical solutions provided in the present invention are as follows:

1) Ionic EC contribution percentage represents the contribution percentage of each ionic activity to EC of nutrient solution, which is calculated by the ionic activity and limit equivalent ionic conductivity:

$$EC_i = \frac{|Z_i| T_i \lambda_\infty}{\sum(|Z_i| T_i \lambda_\infty)} \times 100\%$$

where, $EC_i$ is the ionic EC contribution percentage of ion i, %; $Z_i$ is the charge number carried by ion i; $T_i$ is the ionic activity of ion i, mol/L; and $\lambda\infty$ is the limit equivalent ionic conductivity of ion i, m² S/mol;

2) The ionic activity $T_i$ represents an effective concentration of an ion that plays a role in an electrolyte solution and is a concentration of an ion that plays an electrostatic role in a nutrient solution, which is calculated by ionic activity coefficient and ionic concentration:

$$T_i = \gamma_i C_i$$

where, $T_i$ is the ionic activity of ion i, mol/L; $\gamma_i$ is the ionic activity coefficient of ion i; and $Ci$ is the ionic concentration of ion i, mol/L;

3) The mean ionic activity T represents comprehensive concentration and comprehensive activity of a single salt in a nutrient solution, which is the geometric mean of the ionic activities of cations and anions constituting the single salt in nutrient solution or mixed solution:

$$T = \left(T_i^{v_i} \cdot T_j^{v_j}\right)^{\frac{1}{v_i + v_j}}$$

where, $T$ is the mean ionic activity of a single salt in nutrient solution or a mixed solution, mol/L; $T_i$ and $T_j$ are the ionic activities of the cations and the anions of a single salt in nutrient solution or mixed solution, respectively, mol/L; $v_i$ and $v_j$ are the ion number of the cations and the anions constituting the single salt in nutrient solution or mixed solution, respectively;

4) The ionic activity coefficient $\gamma_i$ is a correction coefficient which represents deviation between an actual solution and an ideal solution, and is a thermodynamic parameter of a solution, which is calculated by Debye-Hückel limiting equation applicable to a nutrient solution:

$$-\lg\gamma_i = \frac{A Z_i^2 \sqrt{I}}{1 + B r_i \sqrt{I}}$$

where, $\gamma_i$ is the ionic activity coefficient of ion i; $A$ is a constant related to temperature, 0.5091 at 25°C; $B$ is a constant related to ionic size, 0.328 at 25°C; $Z_i$ is the charge number carried by ion i; $r_i$ is a volume parameter of ion i; and $I$ is the ionic strength of a solution, mol/L;

$$I = \frac{1}{2} \sum C_i Z_i^2$$

5) The ionic strength I represents electric field strength caused by ions present in a nutrient solution, which is calculated by ionic concentration and charge number carried by each ion constituting the solution:
where, $I$ is the ionic strength of a nutrient solution, mol/L; $C_i$ is the ionic concentration of n i, mol/L; and $Z_i$ is the charge number carried by ion i;

6) Based on mean ionic activity of each single salt in a nutrient solution, the EC of the nutrient solution is estimated by using multivariate linear regression model, and each ionic activity is inversely calculated by using the actually measured EC of nutrient solution and each ionic EC contribution percentage.

Further, the nutrient solution is a nutrient solution formulated based on the horticultural experimental nutrient formula and Yamasaki tomato nutrient formula.

Further, the nutrient solution is prepared in 10 gradients as 0.20, 0.25, 0.33, 0.50, 0.67, 1.00, 1.33, 1.50, 1.80, and 2.00 times of standard concentration.

Further, the ions in the formula refer to the main inorganic ions closely related to plant growth, such as $NO_3^-$, $H_2PO_4^-$, $SO_4^{2-}$, $NH_4^+$, $K^+$, $Ca^{2+}$, $Mg^{2+}$, $Fe^{2+}$, and so on.

Further, methods for actually measuring concentration of each ion in the nutrient solution are described as follows:

Nitrate nitrogen content is measured by spectrophotometer based on colorimetric measurement at 210 nm;

Ammonium nitrogen is measured by spectrophotometer based on indophenol blue colorimetry at 630 nm;

Available phosphorus is measured by spectrophotometer based on the molybdenum blue colorimetry at 680 nm;

Available sulfur is measured by spectrophotometer based on barium sulfate precipitation method at 535 nm;

Available chlorine ion is measured by chromogenic reaction using silver nitrate titrimetry;

Available potassium is measured by atomic absorption spectrophotometer based on flame emission method at 766.5 nm;

Available calcium, magnesium and iron ions are measured by atomic absorption spectrophotometer based on flame absorption method at 422.7 nm, 285.2 nm and 248.3 nm, respectively.

Further, $Ca(NO_3)_2 \cdot 4H_2O$, $KNO_3$, $MgSO_4 \cdot 7H_2O$, $NH_4H_2PO_4$, $K_2SO_4$, $KCl$, and $KH_2PO_4$ single salts with different concentrations are added into each of the nutrient solution respectively. And the concentration of the single salt added into the nutrient solution are set as 0.0, 0.1, 0.2, 0.4, 0.6, 0.8, 1.0, 1.2, 1.4, 1.6, 1.8, and 2.0 times, 12 gradients of concentrations in total.

Further, results of EC of the nutrient solution which is estimated by multivariate linear regression model based on the ionic activity of each ion in the nutrient solution are as follows.

Table 1 Multivariate linear regression model of each ionic activity and EC, pH of nutrient solutions with specific formula

| Formula | EC/ pH | Multivariate linear regression models | $R^2$ |
|---|---|---|---|
| horticultural experimental nutrient formula | EC | $EC=0.02+0.057T_1+0.642T_2+2.135T_3+0.658T_4+0.220T_5-0.329T_6-2.251T_7-0.913T_8$ | 1.0000 |
| | pH | $pH=6.436-0.110T_1-2.568T_2-1.328T_3+5.621T_4+0.292T_5-0.366T_6-0.164T_7-9.569T_8$ | 0.9997 |
| Yamasaki tomato nutrient formula | EC | $EC=0.001+0.172T_1-0.662T_2-0.926T_3-0.570T_4-0.079T_5-0.022T_6+0.738T_7+41.55T_8$ | 0.9999 |
| | pH | $pH=6.468+0.003T_1-0.417T_2-0.849T_3-0.953T_4-0.013T_5+0.191T_6+0.106T_7+33.18T_8$ | 0.9992 |

Where, $T_{1\sim8}$ represent the ionic activities of $NO_3^-$, $H_2PO_4^-$, $SO_4^{2-}$, $K^+$, $Ca^{2+}$, $Mg^{2+}$, $NH_4^+$ and $Fe^{2+}$ in the nutrient solutions, respectively.

The present invention provides a multivariate linear regression model used to estimate EC and pH of a nutrient solution based on the mean ionic activities of main compositions of the nutrient solutions with specific formula. For example, the multivariate linear regression models based on EC, pH and mean ionic activity of compositions in the horticultural experimental nutrient solution and the Yamasaki tomato nutrient solution are as follows.

Table 2 Multivariate linear regression models of EC, pH and mean ionic activity of single salt in compositions of the nutrient solution

| Formula | EC/pH | Multivariate linear regression models | $R^2$ |
|---|---|---|---|
| horticultural experimental nutrient formula | EC | $EC=0.020+0.2861_{KNO3}-0.098T_{Ca(NO3)2.4H2O}+0.671T_{MgSO4}-0.213T_{NH4H2PO4}$ | 0.9997 |
| | pH | $pH=6.376-0.170T_{KNO3}-0.027T_{Ca(NO3)2.4H2O}+0,419T_{MgSO4}+0.20T_{NH4H2PO4}$ | 0.9910 |
| Yamasaki tomato nutrient formula | EC | $EC=0.013-0.270_{KNO3}+0.846T_{Ca(NO3)2·4H2OHUO}+0.256T_{MgSO4}+0.1061_{NH4H2PO4}$ | 0.9997 |
| | pH | $pH=6.484-0.105T_{KNO3}+0.165T_{Ca(NO3)2·4H2O}-0.161T_{MgSO4}-0.139T_{NH4H2}$ | 0.9997 |

The present invention also clarifies that the effect of the mean ionic activity of a specific single salt added in nutrient solution on EC is linear. For example, the linear regression models of the mean ionic activity and EC of the horticultural experimental nutrient solution and the Yamasaki tomato nutrient solution after a specific single salt added are as follows.

Table 3 Effect of mean ionic activity of the specific single salt added on EC of the nutrient solution

| Single salts added | horticultural experimental nutrient solution | | Yamasaki tomato nutrient solution | |
|---|---|---|---|---|
| | Linear regression equations | $R^2$ | Linear regression equations | $R^2$ |
| $K_2SO_4$ | $EC=0.156Ti+2.321$ | 0.9996 | $EC=0.168T_i+1.154$ | 0.9992 |
| $KNO_3$ | $EC=0.145T_i+2.317$ | 0.9989 | $EC=0.1487T_i+1.157$ | 0.9995 |
| $KCl$ | $EC=0.156Ti+2.320$ | 0.9994 | $EC=0.151T_i+1.164$ | 0.9992 |
| $KH_2PO_4$ | $EC=0.101Ti+2.334$ | 0.999 | $EC=0.1067T_i+1.163$ | 0.9978 |
| $Ca(NO_3)_2 \cdot 4H_2O$ | $EC=0.145T_i+2.318$ | 0.9972 | $EC=0.1477T_i+1.159$ | 0.9994 |
| $MgSO_4 \cdot 7H_2O$ | $EC=0.159Ti+2.329$ | 0.9977 | $EC=0.1777T_i+1.166$ | 0.9995 |
| $NH_4H_2PO_4$ | $EC=0.102T_i+2.332$ | 0.9957 | $EC=0.1047T_i+1.163$ | 0.9986 |

The present invention also provides multivariate linear regression models of the mean ionic activity of each main composition in a nutrient solution after a specific single salt added and EC of the nutrient solution, as follows.

Table 4 Multivariate linear regression models of mean ionic activity and EC of a nutrient solution after a specific single salt added

| Formula | Single salt added | Multivariate linear regression models | $R^2$ |
|---|---|---|---|
| | | | |
| horticultural experimental nutrient solution | $K_2SO_4$ | $EC=340.9-0.0863T_x=111.8T_{Ca(NO3)2}-0.0807T_{KNO3}-0.8521T_{MgS}+97.49\ T_{NH4H2PO4}$ | 0.9996 |
| | $KNO_3$ | $EC=1508+0.0847T_x-0.2729T_{Ca(NO3)2}-0.1808T_{KNO3}-1451\ T_{Mg}+369.4T_{NH4H2PO4}$ | 0.9996 |
| | $KCl$ | $EC=-7731+2.5254T_x+1656T_{Ca(NO3)2}+2.2094T_{KNO3}+2550\ T_{MgSO4}-2060T_{NH4H2PO4}$ | 0.9996 |
| | $KH_2PO_4$ | $EC=-386.6-0.6181T_x-704T_{Ca(NO3)2}-0.8554T_{KNO3}+2419\ T_{MgSO4}-0.2049T_{NH4H2PO4}$ | 0.9991 |
| | $Ca(NO_3)_2$ | $EC=1422-0.0119T_x-0.578lT_{Ca(NO3)2}+0.2657T_{KNO3}-1358\ T_{MgS}+341.5T_{NH4H2PO4}$ | 0.9999 |
| | $MgSO_4$ | $EC=-9064+0.0094T_x+8507T_{Ca(NO3)2}-4562T_{KNO3}-0.263\ T_{MgSO4}-2130T_{NH4H2PO4}$ | 0.9972 |
| | $NH_4H_2PO_4$ | $EC=478.1+1.0418T_x-1841T_{Ca(NO3)2}+1394T_{KNO3}+OT_{MgSO4}+0.872T_{NH4H2PO4}$ | 0.9966 |
| Yamasaki tomato nutrient solution | $K_2SO_4$ | $EC=84.57+0.1481T_x-74.45T_{Ca(NO3)2}-0.1214T_{KNO3}+0.262T+112.6T_{NH4H2PO4}$ | |
| | $KNO_3$ | $EC=66.59+0.1184T_x+0.1721T_{Ca(NO3)2}-0.2016T_{KNO3}-118T_{MgS}+57.04T_{NH4H2PO4}$ | 0.9999 |
| | $KCl$ | $EC=959.4+0.131T_x-27.5T_{Ca(NO3)2}-0.2202T_{KNO3}-1824T_{MgSO4}+1036T_{NH4H2PO4}$ | 0.9993 |
| | $KH_2PO_4$ | $EC=28.84+0.2328T_x+213.2T_{Ca(NO3)2}-0.06337_{KNO3}-552.1T_{MgSO4}-0.26\ NH_4H_2PO_4$ | 0.999 8 |

(continued)

| Formula | Single salt added | Multivariate linear regression models | $R^2$ |
|---|---|---|---|
| | $Ca(NO_3)_2$ | $EC=494.78+0.1804T_x-0.08237_{Ca(NO3)2}+ 0.0721T_{KNO3}-978.5T_{MgSO4}+538.3T_{NH4H2PO4}$ | 0.999 8 |
| | $MgSO_4$ | $EC=-2.6905+0.0078T_x+ 107.3T_{Ca(NO3)2}-63.58T_{KNO3}-0.289T_{MgnSO4}+0.0961T_{NH4H2PO4}$ | 0.999 9 |
| | $NH_4H_2PO_4$ | $EC=-121.9+0.12577_x+0T_{Ca(NO3)2}-158T_{KNO3}+ 779.4T_{MgSO4}-0.0891T_{NH4H2PO4}$ | 0.999 1 |

Note: $T_x$ in the table represents the mean ionic activity of the specific single salt added.

## Beneficial effects of the present invention

The technical core of the present invention lies in a method for calculating ionic EC contribution percentage and electrical conductivity of a nutrient solution with a specific formula based on ionic activity, which could be used for management of protected cultivation based on a nutrient solution formula and can upgrade the nutrient solution management in the protected cultivation from an EC level to an ionic concentration level. The increase of yield for tomato and strawberry are over 15% and 20%, respectively. The benefit increased is calculated as 25000 to 40000 $CNY/hm^2$ by the yield-increasing effect at a nutrient solution intelligent management level.

The technical core of the present invention lies in a method for calculating ionic EC contribution percentage and electrical conductivity of a nutrient solution with a specific formula based on an ionic activity, which can be applied for any management of protected cultivation based on nutrient solution formula. Therefore, it could be applied and popularized not only in the development of nutrient solution regulation and control system, but also in the intelligent matching between EC and pH of a nutrient solution and each ionic concentration of different crops at different growth stages.

The method of the present invention can not only be used to calculate the electrical conductivity and potential of hydrogen of a nutrient solution with a specific formula, but may also inversely calculate each ionic concentration of a nutrient solution using actual measurement of electrical conductivity in combination each ionic EC contribution percentage, providing a theoretical basis and technical support for dynamic regulation and control that is based on the ionic concentration and difficult to implement for the nutrient solution in soilless culture and integration of water and fertilizer. The method is simple, easy to implement, and very accurate. The method may be used for developing nutrient solution regulation and control systems, thereby achieving automatic control of smart matching demands for the established EC and pH of a nutrient solution and each ionic concentration by different crops at different growth stages and formula adjustment.

## BRIEF DESCRIPTION OF THE DRAWINGS

FIG. 1 is a diagram of changes of ionic EC contribution percentage of each ion in a nutrient solution having a specific formula of different concentrations of the present invention;

FIG. 2 is a diagram of comparison between estimated EC and measured EC in a nutrient solutions having a specific formula of the present invention; and

FIG. 3 is a diagram of a response of the ionic EC contribution percentage of each ion in the horticultural experimental nutrient solution to the added specific single salt.

## DETAILED DESCRIPTION

For a problem that concentration of each inorganic ion is difficult to be reflected by regulation and control of EC and pH of existing nutrient solution irrigation system, the characteristic of the present invention lies in, a method for calculating an ionic EC contribution percentage and EC of a nutrient solution based on ionic activity, and explore a new method for inversely calculating each ionic concentration in a nutrient solution using the ionic EC contribution percentage and actual measured EC, thereby providing a theoretical basis and a technical support for realizing feedback regulation and control of ionic concentration in a nutrient solution. In order to test the correctness of the theory and the solution, the present invention establishes a multivariate linear regression model based on ionic EC contribution percentage calculation of ionic activity and EC in view of horticultural experimental nutrient formula and Yamasaki tomato nutrient formula. The method of the present invention is suitable for various nutrient solution formula, and not limited to the horticultural experimental nutrient formula and the Yamasaki tomato nutrient formula. The following embodiments are used to illustrate technical method of the present invention, but not limited to the scope of the present invention. Unless specifically indicated, the technical method used in the embodiments is conventional technical method which are well known by a technician skilled in the field, and the materials used are commercially available product.

Example 1 Calculation of ionic EC contribution percentage and EC of a nutrient solution with a specific formula

According to different demands of crops for a concentration of a nutrient solution at different growth stages, HENS and YTNS are prepared in 10 gradients as 0.20, 0.25, 0.33, 0.50, 0.67, 1.00, 1.33, 1.50, 1.80, and 2.00 times of standard concentration. The ionic EC contribution percentage of each inorganic ion and EC of nutrient solutionare calculated according to the method of the present invention. A nutrient solution to be tested is prepared with analytic pure reagents of $Ca(NO_3)_2 \cdot 4H_2O$, $KNO_3$, $MgSO_4 \cdot 7H_2O$, $NH_4H_2PO_4$, $MnSO_4 \cdot H_2O$, $CuSO_4 \cdot 5H_2O$, $ZnSO_4 \cdot 7H_2O$, $H_3BO_3$, $(NH_4)_6Mo_7O_{24} \cdot 4H_2O$ (Xilong chemical reagents co. , Guangdong), and distilled water with EC value of $11 \pm 2$ $\mu$S/cm and pH of $5.82 \pm 0.08$. DTPA-Fe-7 (Shanghai yongtong chemical co., LTD., Shanghai) with 7% EDTA-Fe is used as chelated iron. Analytic pure reagents of $K_2SO_4$, $KCl$, and $KH_2PO_4$ are added in $K^+$ solution for measurement of ionic EC contribution percentage.

A portable multi-parameter analyzer (HQ-40d, HACH Co., Ltd., USA) is used to actually measure the EC and pH of the nutrient solution to be detected. The measurement methods for ionic concentration of each ion I in the nutrient solution are as follows: nitrate nitrogen conten is measured by spectrophotometer (UV-3150, Shimadzu Co., Ltd., Japan) based on colorimetric measurement at 210 nm (HJ/T346-2007); ammonium nitrogen is measured by spectrophotometer based on indophenol blue colorimetry at 630 nm (HJ/T537-2009); available phosphorus groupis measured by spectrophotometer based on the molybdenum blue colorimetry at 680 nm(HJ/T593-2010); available sulfur group is measured by spectrophotometer based on barium sulfate precipitation method at 535 nm for turbidity (HJ/T342-2007); available chlorine ion is measured by chromogenic reaction using silver nitrate titrimetry (GBT11896-89); available potassium ion is measured by atomic absorption spectrophotometer (AA-7002, Beijing East and West Analytical Instrument Co., Beijing) based on flame emission method at 766.5 nm (GB11904-1989); and available calcium, magnesium (GB11905-1989) and iron ions (GB11911-1989) are measured by atomic absorption spectrophotometer based on flame absorption method at 422.7 nm, 285.2 nm and 248.3 nm, respectively.

Table 5 Japanese horticultural experimental nutrient formula and Yamasaki tomato nutrient formula commonly used for soilless culture

| Macro-element | horticultural experimental nutrient formula | Yamasaki tomato nutrient formula | Micro-element | horticultural experimental nutrient formula | Yamasaki tomato nutrient formula |
|---|---|---|---|---|---|
| | mmol/L | mmol/L | | mg/L | mg/L |
| $Ca(NO_3)_2 \cdot 4H_2O$ | 4 | 1.5 | Fe-DTPA(7%) | 42.857 | 28.571 |
| $KNO_3$ | 8 | 4 | $MnSO_4 \cdot H_2O$ | 1.538 | 0.615 |
| $MgSO_4 \cdot 7H_2O$ | 2 | 1 | $CuSO_4 \cdot 5H_2O$ | 0.078 | 0.039 |
| $NH_4H_2PO_4$ | 1.33 | 0.67 | $ZnSO_4 \cdot 7H_2O$ | 0.220 | 0.088 |
| - | - | - | $H_3BO_3$ | 2.818 | 1.127 |
| - | - | - | $(NH_4)_6Mo_7O_{24} \cdot 4H_2O$ | 0.026 | 0.013 |

For nutrient solutions of each of concentration gradients, corresponding contents of reagents are weighed accurately (accurate to 1.0 mg) and sufficiently dissolved, then mixed, After addition of micro-elements with corresponding concentration gradient, 1L of the mixed solution is formulated, which is stored in a dark condition after shaken well, as a nutrient solution to be tested.

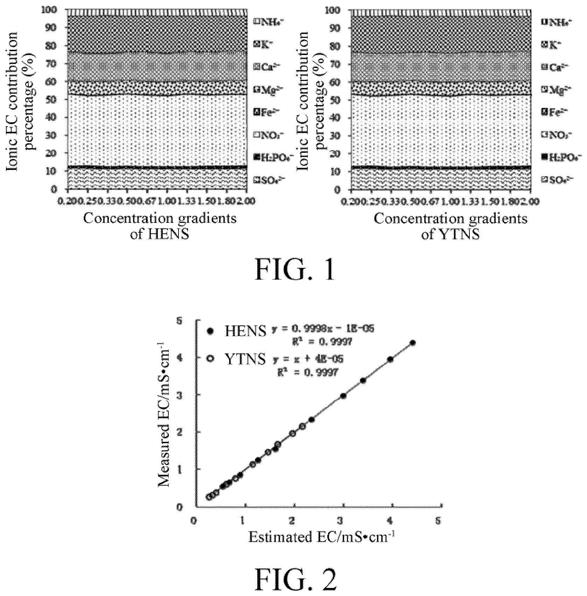

When the concentration of nutrient solution having a specific formula ranges from 0 to 2.0 times of the standard concentration, the change of the ionic EC contribution percentage of each ion in the nutrient solution based on horticultural experimental nutrient formula is 2% or less except iron, only the change of the ionic EC contribution percentage of $Fe^{2+}$ is 5% or less; the change of the ionic EC contribution percentage of each ion in the nutrient solution based on Yamasaki tomato nutrient formula is 2% or less except $H_2PO_4^-$ (4%) and $Fe^{2+}$ (6%) (FIG. 1). The change of the theoretically calculated ionic EC contribution percentage of each ion within a range of concentration in the nutrient solutions is about 1%. The difference between the theoretically calculated values and the actually measured values is due to that composition of nutrient solution or reagents actually used may not be able to dissolve completely or not reach the boundary condition of Debye-Hückel limting equation. The ionic EC contribution percentage of each of ions in mixed solutions are also different because of the differences in ionic electro-conductivity among ions, which is resulted from difference of ionic concentrations, charge number and ion radius of each ion in the nutrient solution. Because the nutrient solution used in crop culture is a relatively dilute electrolyte solution and a variation range of concentration is limited, a variation range of the ionic EC contribution percentage of the nutrient solution with a certain concentration range is relatively small. Therefore, when the concentration of a nutrient solution with a specific formula is within a certain range, changes of the EC contribution percentage of each ion to EC thereof along with changes of relative concentration of the nutrient solution are neglectable. The result indicates that each ionic concentration of the nutrient solution with a specific formula could be quantitatively figured out by using actually measured EC based on the characteristic that the ionic EC contribution percentage is relatively stable.

Estimated EC value are calculated by multivariate linear regression models of EC and mean ion activities of compositions in the horticultural experimental nutrient solution (HENS) and Yamasaki tomato nutrient solution (YTNS) with different concentration. The relative errors between estimated EC and measured EC are 1.4% in HENS and 1.8% in YTNS (FIG. 2). Therefore, EC of the nutrient solution can be accurately estimated by using the mean ionic activities of single salt in the nutrient solution based on the multivariate linear regression models mentioned above,. And each ionic activity is inversely calculated by using the actually measured EC and each ionic EC contribution percentage.

Example 2 Changes of an ionic EC contribution percentage of a nutrient solution after a specific single salt added

To test changes of ionic EC contribution percentage of main ion compositions in a mixed solution after $Ca(NO_3)_2 \cdot 4H_2O$, $KNO_3$, $MgSO_4 \cdot 7H_2O$, $NH_4H_2PO_4$, $K_2SO_4$, KCl and $KH_2PO_4$ with 12 different concentration gradients are separately added to HENS and YTNS, respectively, the addition amount of each single salt is set as 0.0, 0.1, 0.2, 0.4 , 0.6, 0.8, 1.0, 1.2, 1.4, 1.6, 1.8, and 2.0 times of standard concentration ( with the formula ionic concentration as a standard). After 100 L standard nutrient solutions are prepared based on HENS and YTNS, the single salts in foregoing concentration gradients are separately added to the nutrient solutions ( less than 800 ml) and then fully mixed, and the constant volume of the solution is 1 L. The solutions to be tested are stored in a dark condition after shaken well.

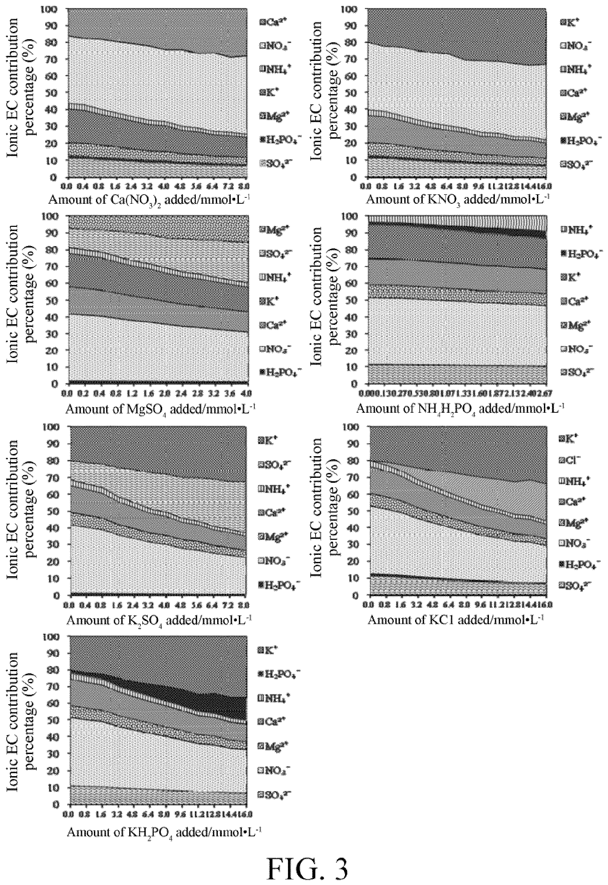

When specific single salts with 12 different concentration gradients are added respectively into HENS and YTNS, the ionic EC contribution percentage of each ion of the specific single salt is increased to some extent, while the ionic EC contribution percentage of other ions decreased correspondingly (FIG. 3). When $Ca(NO_3)_2 \cdot 4H_2O$, $KNO_3$, $MgSO_4 \cdot 7H_2O$ and $NH_4H_2PO_4$ that have been in a nutrient solution are added separately in the nutrient solution, minimum of relative change of each ionic EC contribution percentage is $NH_4H_2PO_4$. The increasing changes of the ionic EC contribution percentage of cations and anions in $MgSO_4 \cdot 7H_2O$ and $NH_4H_2PO_4$ in nutrient solution show a similar trend, but increasing changes of the ionic EC contribution percentage of cations are 5-6 times that of anions in $Ca(NO_3)_2 \cdot 4H_2O$ and $KNO_3$ in nutrient solution after addition. The results above indicate that different ions have greatly different effects on its ionic EC contribution percentage due to characteristics of ion itself, which provides a theoretical basis for distinguishing the ways to add or regulate specific ion in a process of dynamic regulation and control of a nutrient solution.

When $KNO_3$, $K_2SO_4$, KCl and $KH_2PO_4$ are separately added with same $K^+$ concentration, changes of the ionic EC contribution percentage of $K^+$ are similar, while ionic EC contribution percentages of different anions are different, with $Cl^-$ having the maximum relative change of the EC contribution percentage of anions, followed by $H_2PO_4^-$, and relative change of the ionic EC contribution percentage of $NO_3^-$ is smallest. The results indicate that there is no short interaction between ions; the ionic EC contribution percentage is related only to the ionic activity.

By comparison of the estimated EC of the nutrient solution after a specific single salt added by using the foregoing multivariate linear regression models and the actually measured EC, The result obtained shows that the relative deviation between the estimated EC value and the actually measured EC value are respectively 0.10%-0.28% in HENS and 0.03%-0.33% in YTNS after the specific single salt is added (Table 6).

Therefore, although there are some differences in the ionic EC contribution percentage based on a nutrient solution with a specific formula, the ionic concentration of each ion in the nutrient solution could be fully figured out by calculation of the ionic EC contribution percentage of the single salt added and the actual measured EC, which provides a theoretical basis and a technical support for achieving the dynamic regulation and control of ionic concentration level based on the actual measured EC of a nutrient solution.

Table 6 Comparison between estimated EC and actually measured EC in the nutrient solutions after a specific single salt added

| Single salt added | Estimated error of EC in HENS | | | Estimated error of EC in YTNS | | |
|---|---|---|---|---|---|---|
| | Linear regression equation | $R^2$ | Relative error/% | regression equation | $R^2$ | Relative error/% |
| $Ca(NO_3)_2$ | y=x-4E-0 | 0.9999 | 0.10±0.08 | y=x-3E-0 | 0.9999 | 0.14±0.09 |
| $KNO_3$ | y=x | 1.0000 | 0.27±0.21 | y=x-3E-05 | 0.9999 | 0.14±0.08 |
| $MgSO_4$ | y=x+6E-06 | 0.9985 | 0.23±0.12 | y=x+5E-06 | 1.0000 | 0.03±0.02 |
| $NH_4H_2PO_4$ | y=x+3E-1 | 0.9969 | 0.15±0.09 | y=x-5E-13 | 0.9991 | 0.07±0.07 |
| $K_2SO_4$ | y=x-3E-05 | 0.9998 | 0.21±0.19 | y=x-4E-05 | 0.9999 | 0.13±0.11 |
| KCl | y=x-3E-05 | 0.9998 | 0.26±0.20 | y=x+3E-05 | 0.9996 | 0.33±0.26 |
| $KH_2PO_4$ | y=x+2E-05 | 0.9995 | 0.28±0.18 | y=x+5E-05 | 0.9999 | 0.13±0.11 |

## Claims

1. A method for calculating an ionic electrical conductivity (EC) contribution percentage and electrical conductivity of a nutrient solution based on an ionic activity, **characterized in that** the method comprises the following steps:

   1) calculating the ionic EC contribution percentage that is proposed and used to represent the contribution percentage of each ionic activity to the EC in the nutrient solution:

$$EC_i = \frac{|Z_i| T_i \lambda_\infty}{\sum(|Z_i| T_i \lambda_\infty)} \times 100\%$$

   wherein, $EC_i$ is the ionic EC contribution percentage of ion i, %; $Z_i$ is the charge number carried by the ion i; $T_i$ is the ionic activity of the ion i, mol/L; and $\lambda_\infty$ is the limit equivalent ionic conductivity of the ion i, $m^2$ S/mol;
   2) calculating the ionic activity which represents an effective concentration of the ion that plays a role in electrolyte solution and is a concentration of an ion that plays an electrostatic role in the nutrient solution:

$$T_i = \gamma_i C_i$$

   wherein, $T_i$ is the ionic activity of the ion i, mol/L; $\gamma_i$ is the ionic activity coefficient of the ion i; $Ci$ is the ionic concentration of the ion i, mol/L;
   3) calculating the mean ionic activity that represents comprehensive concentration or comprehensive activity of the ions in a single salt solution or a mixed solution:

$$T = \left(T_i^{v_i} \cdot T_j^{v_j}\right)^{\frac{1}{v_i + v_j}}$$

   wherein, $T$ is the mean ionic activity of a single salt solution or a mixed solution, mol/L; $T_i$ and $T_j$ respectively are the ionic activities of the cations and the anions constituting the single salt solution or the mixed solution, mol/L; $v_i$ and $v_j$ respectively are the number of the cations and the anions constituting the single salt solution or the mixed solution;
   4) calculating, by Debye-Hiickel limiting equation applicable to characteristics of the nutrient solution, the ionic activity coefficient that is a correction coefficient representing deviation between an actual solution and an ideal solution:

$$-\lg\gamma_i = \frac{AZ_i^2\sqrt{I}}{1 + Br_i\sqrt{I}}$$

wherein, $y_i$ is the ionic activity coefficient of the ion i; $A$ is a constant related to temperature, 0.5091 at 25°C; $B$ is a constant related to ionic size, 0.328 at 25°C; $Z_i$ is the charge number carried by the ion i; $r_i$ is a volume parameter of the ion i; $I$ is ionic strength of the nutrient solution, mol/L;

5) calculating the ionic strength that represents the electric field strength caused by the ions present in the nutrient solution:

$$I = \frac{1}{2}\sum C_i Z_i^2$$

wherein, $I$ is the ionic strength of the nutrient solution, mol/L; $C_i$ is the ionic concentration of ion i, mol/L; $Z_i$ is the charge number carried by the ion i;

6) measuring the EC and pH of the nutrient solution;

7) based on the mean ionic activities $T$ of each single salt in the nutrient solution, estimating the EC of the nutrient solution by multivariate linear regression models, and inversely calculating the ionic concentration by using the actually measured EC and ionic EC contribution percentage.

**2.** The method according to claim 1, **characterized in that**, the ions in the nutrient solution are selected from the group consisting of main inorganic ions $NO_3^-$, $H_2PO_4^-$, $SO_4^{2-}$, $NH_4^+$, $K^+$, $Ca^{2+}$, $Mg^{2+}$ and $Fe^{2+}$ which are closely related to plant growth, the multivariate linear regression models of EC of the nutrient solution with the specific formula are established based on the ionic activity of the foregoing main inorganic ions; and the multivariate linear regression models of each ionic activity and EC of the nutrient solution established based on a horticultural experimental nutrient formula and a Yamasaki tomato nutrient formula are as follows:

| Formula | EC/pH | Multivariate linear regression models | $R^2$ |
|---|---|---|---|
| horticultural experimenta I nutrient formula | EC | EC= $0.02+0.057T_i+0.642T_2+2.135T_3+0.658T_4+0.22OT_5-0.329T_6-2.251T_7-0.913T_8$ | 1.0000 |
| Yamasaki tomato nutrient formula | EC | EC= $0.001+0.172T_1-0.662T_2-0.926T_3-0.570T_4-0.079T_5-0.022T_6+0.738T_7+41.55T_8$ | 0.9999 |

wherein $T_{1\sim8}$ represent the ionic activities of $NO_3^-$, $H_2PO_4^-$, $SO_4^{2-}$, $K^+$, $Ca^{2+}$, $Mg^{2+}$ $NH_4^+$, $Fe^{2+}$ in the nutrient solutions, respectively.

**3.** The method according to claim 1 or 2, **characterized in that**, the multivariate linear regression equations of EC of the nutrient solution is estimated using the mean ionic activities $T$ of the main compositions of the nutrient solution with the specific formula; and the multivariate linear regression models based on the mean ionic activities of compositions in the horticultural experimental nutrient solution and the Yamasaki tomato nutrient solution and their EC are as follows:

| Formula | EC/pH | Multivariate linear regression equations | $R^2$ |
|---|---|---|---|
| horticultural experimental nutrient formula | EC | EC = $0.020+0.286T_{KNO3}-0.098T_{Ca(NO3)2·4H2O}+0.671T_{MgSO4}-0.213T_{NH4H2PO4}$ | 0.9997 |

(continued)

| Formula | EC/pH | Multivariate linear regression equations | $R^2$ |
|---|---|---|---|
| tomato nutrient formula | EC | EC = $0.013-0.270T_{KNO3}+0.846T_{Ca(NO3)2\cdot4H2O}+0.256T_{MgSO4}+0.106T_{NH4H2PO4}$ | 0.9997 |

**4.** The method according to claim 1 or 2, **characterized in that**, the linear regression models of the mean ionic activity $T$ and EC in the nutrient solution with the specific formula after addition of a specific single salt are used to estimate the EC; the multivariate linear regression models based on the mean ionic activity of the specific single salt added and their EC in the horticultural experimental nutrient solution (HENS) and Yamasaki tomato nutrient solution (YTNS) are as follows:

| Single salts added | HENS | | YTNS | |
|---|---|---|---|---|
| | Linear regression equations | $R^2$ | Linear regression equations | $R^2$ |
| $K_2SO_4$ | EC=$0.156T_i$+2.321 | 0.9996 | EC=$0.168T_i$+1.154 | 0.9992 |
| $KNO_3$ | EC=$0.145T_i$+2.317 | 0.9989 | EC=$0.148T_i$+1.157 | 0.9995 |
| KCl | EC=$0.156T_i$+2.320 | 0.9994 | EC=$0.151T_i$+1.164 | 0.9992 |
| $KH_2PO_4$ | EC=$0.101T_i$+2.334 | 0.999 | EC=$0.106T_i$+1.163 | 0.9978 |
| $Ca(NO_3)_2\cdot4H_2O$ | EC=$0.145T_i$+2.318 | 0.9972 | EC=$0.147T_i$+1.159 | 0.9994 |
| $MgSO_4\cdot7H_2O$ | EC=$0.159T_i$+2.329 | 0.9977 | EC=$0.177T_i$+1.166 | 0.9995 |
| $NH_4H_2PO_4$ | EC=$0.102T_i$+2.332 | 0.9957 | EC=$0.104T_i$+1.163 | 0.9986 |

**5.** The method according to claim 1 or 2, **characterized in that**, the multivariate linear regression models are established based on the mean ionic activity $T$ of the specific single salt added and the mean ionic activity $T$ of other single salts in the nutrient solution with the specific formula and their EC; and the multivariate linear regression models based on the mean ionic activity of the specific single salt added and the mean ionic activity of other compositions in the horticultural experimental nutrient solution (HENS) and Yamasaki tomato nutrient solution (YTNS) and their EC are as follows:

| Formula | Single salts added | Multivariate linear regression models | $R^2$ |
|---|---|---|---|
| HENS | $K_2SO_4$ | EC=$340.9-0.0863T_x-111.8T_{Ca(NO3)2}-0.0807T_{KNO3}-0.8521T_{MgSO4}+97.49T_{NH4H2PO4}$ | 0.9996 |
| | $KNO_3$ | EC=$1508+0.0847T_x-0.2729T_{Ca(NO3)2}-0.1808T_{KNO3}-1451T_{MgSO4}+369.4T_{NH4H2PO4}$ | 0.9996 |
| | KCl | EC=$-7731+2.5254T_x+1656T_{Ca(NO3)2}+2.2094T_{KNO3}+2550T_{MgSO4}-2060T_{NH4H2PO4}$ | 0.9996 |
| | $KH_2PO_4$ | EC=$-386.6-0.6181T_x-704T_{Ca(NO3)2}-0.8554T_{KNO3}+2419T_{Mgso4}-0.2049T_{NH4H2PO4}$ | 0.9991 |
| | $Ca(NO_3)_2$ | EC=$1422-0.0119T_x-0.5781T_{Ca(NO3)2}+0.2657T_{KNO3}-1358T_{MgSO4}+341.5T_{NH4H2PO4}$ | 0.9999 |
| | $MgSO_4$ | EC=$-9064+0.0094T_x+8507T_{Ca(NO3)2}-4562T_{KMO3}-0.263T_{Mgso4}-2130T_{NH4H2PO4}$ | 0.9972 |
| | $NH_4H_2PO_4$ | EC=$478.1+1.0418T_x-1841T_{Ca(NO3)2}+1394T_{KNO3}-0T_{MgSO4}+0.872T_{NH4H2PO4}$ | 0.9969 |

(continued)

| Formula | Single salts added | Multivariate linear regression models | $R^2$ |
|---|---|---|---|
| YTNS | $K_2SO_4$ | $EC=84.57+0.1481T_x-74.45T_{Ca(NO3)2}-0.1214T_{KNO3}+0.262T_{MgSO4}+112.6T_{NH4H2PO4}$ | 0.9998 |
| | $KNO_3$ | $EC=66.59+0.1184T_x+0.1721T_{Ca(NO3)2}-0.2016T_{KNO3}-118T_{MgSO4}+57.04T_{NH4H2PO4}$ | 0.9999 |
| | $KCl$ | $EC=959.4+0.131T_x-27.5T_{Ca(NO3)2}-0.2202T_{KNO3}-1824T_{MgSO4}+1036T_{NH4H2PO4}$ | 0.9993 |
| | $KH_2PO_4$ | $EC=28.84+0.2328T_x+213.2T_{Ca(NO3)2}-0.0633T_{KNO3}-552.1T_{MgSO4}-0.2662T_{NH4H2PO4}$ | 0.9998 |
| | $Ca(NO_3)_2$ | $EC=494.78+0.1804T.-0.0823T_{Ca(NO3)2}\ 0.0721T_{KNO3}-978.5T_{Mgso4}+538.3T_{NH4H2PO4}$ | 0.9998 |
| | $MgSO_4$ | $EC=-2.6905+0.0078T_x+107.3T_{Ca(NO3)2}-63.58T_{KNO3}-0.289T_{MgSO4}+0.0961T_{NH4H2PO4}$ | 0.9999 |
| | $NH_4H_2PO_4$ | $EC=-121.9+0.1257T_x+0T_{Ca(NO3)2}-158T_{KNO3}+779.4T_{MgSO4}-0.0891T_{NH4H2PO4}$ | 0.9991 |

Note: $T_x$ represents the mean ionic activity of the specific single salt added.

6. The method according to claim 1 or 2, **characterized in that**, the nutrient solutions for test in the method are nutrient solutions prepared based on the horticultural experimental nutrient solution formula and the Yamasaki tomato nutrient solution formula.

**Patentansprüche**

1. Verfahren zum Berechnen eines prozentualen Beitrags einer ionischen elektrischen Leitfähigkeit (EC) und einer elektrischen Leitfähigkeit einer Nährlösung auf der Grundlage einer ionischen Aktivität, **dadurch gekennzeichnet, dass** das Verfahren die folgenden Schritte umfasst:

1) berechnen des Prozentsatzes des ionischen EC-Beitrags, der vorgeschlagen und verwendet wird, um den Prozentsatz des Beitrags jeder ionischen Aktivität zum EC in der Nährlösung darzustellen:

$$EC_i = \frac{|Z_i|\, T_i \lambda_\infty}{\sum(|Z_i|\, T_i \lambda_\infty)} \times 100\%$$

wobei, $EC_i$ ist der prozentuale Anteil des ionischen EC-Beitrags von Ion i, %; $Z_i$ ist die Ladungszahl, die das Ion i trägt; $T_i$ ist die ionische Aktivität des Ions i, mol/L; und $\lambda_\infty$ ist die Grenzäquivalent-Ionenleitfähigkeit des Ions i, $m^2$ S/mol;

2) berechnen der ionischen Aktivität, die eine effektive Konzentration des Ions darstellt, das eine Rolle in der Elektrolytlösung spielt, und eine Konzentration eines Ions ist, das eine elektrostatische Rolle in der Nährlösung spielt:

$$T_i = \gamma_i C_i$$

wobei, $T_i$ ist die Ionenaktivität des Ions i, mol/L; $\gamma_i$ ist der Ionenaktivitätskoeffizient des Ions i; $C_i$ ist die Ionen-konzentration des Ions i, mol/L;

3) berechnen der mittleren ionischen Aktivität, die die die Gesamtkonzentration oder Gesamtaktivität der Ionen in einer Einzelsalzlösung oder einer Mischlösung darstellt:

$$T = \left(T_i{}^{v_i} \cdot T_j{}^{v_j}\right)^{\frac{1}{v_i+v_j}}$$

wobei, $T$ ist die mittlere ionische Aktivität einer Einzelsalzlösung oder einer Mischlösung, mol/L,; $T_i$ bzw. $T_j$ sind die ionischen Aktivitäten der Kationen und der Anionen, die die Einzelsalzlösung oder die Mischlösung bilden, mol/L; $v_i$ bzw. $v_j$ sind die Anzahl der Kationen und der Anionen, aus denen die Einzelsalzlösung oder die Mischlösung besteht;

4) berechnen des Ionenaktivitätskoeffizienten, der ein Korrekturkoeffizient ist, der die Abweichung zwischen einer tatsächlichen Lösung und einer idealen Lösung darstellt, mit der auf die Eigenschaften der Nährlösung anwendbaren Debye-Hückel-Grenzgleichung:

$$-\lg\gamma_i = \frac{A Z_i{}^2 \sqrt{I}}{1 + B r_i \sqrt{I}}$$

wobei, $\gamma_i$ ist der ionische Aktivitätskoeffizient des Ions i; $A$ ist eine Konstante, die sich auf die Temperatur bezieht, 0,5091 bei 25°C; $B$ ist eine Konstante, die sich auf die Ionengröße bezieht, 0,328 bei 25°C; $Z$, ist die Ladungszahl, die das Ion i trägt; $r_i$ ist ein Volumenparameter des Ions i; $I$ ist die Ionenstärke der Nährlösung, mol/L;

5) berechnen der Ionenstärke, die die elektrische Feldstärke darstellt, die durch die in der Nährlösung vorhandenen Ionen verursacht wird:

$$I = \frac{1}{2} \sum C_i Z_i{}^2$$

wobei, $I$ ist die Ionenstärke der Nährlösung, mol/L; $C_i$ ist die Ionenkonzentration des Ions i, mol/L; $Z_i$ ist die Ladungszahl, die das Ion i trägt;

6) messen des EC- und pH-Werts der Nährlösung;

7) basierend auf der mittleren ionischen Aktivität $T$ jedes einzelnen Salzes in der Nährlösung, Schätzung des EC der Nährlösung durch multivariate lineare Regressionsmodelle und inverse Berechnung der Ionenkonzentration unter Verwendung des tatsächlich gemessenen EC- und ionischen EC-Beitragsprozentsatzes.

2. Verfahren nach Anspruch 1, **dadurch gekennzeichnet, dass** die Ionen in der Nährlösung ausgewählt sind aus der Gruppe bestehend aus den wichtigsten anorganischen Ionen $NO_3^-$, $H_2PO_4^-$; $SO_4^{2-}$, $NH_4^+$, $K^+$, $Ca^{2+}$, $Mg^{2+}$ und $Fe^{2+}$, die eng mit dem Pflanzenwachstum verbunden sind, die multivariaten linearen Regressionsmodelle für den EC-Wert der Nährlösung mit der spezifischen Formel werden auf der Grundlage der Ionenaktivität der vorgenannten wichtigsten anorganischen Ionen erstellt; und die multivariaten linearen Regressionsmodelle für jede Ionenaktivität und den EC-Wert der Nährlösung, die auf der Grundlage einer experimentellen Nährstoffformel für den Gartenbau und einer Yamasaki-Nährstoffformel für Tomaten erstellt wurden, lauten wie folgt:

| Formel | EC/ pH | Multivariate lineare Regressionsmodelle | $R^2$ |
|---|---|---|---|
| Experimente lle Nährstofffor mel für den Gartenbau | EC | EC= $0,02+0,057T_1+0,642T_2+2,135T_3+0,658T_4+0,220T_5-0,329T_6-2,251T_7-0,913T_8$ | 1,0000 |
| Yamasaki Tomaten- Nährstofffor mel | EC | EC= $0,001+0,172T_1-0,662T_2-0,926T_3-0,570T_4-0,079T_5-0,022T_6+0,738T_7+41,55T_8$ | 0,9999 |

wobei $T_{1\sim8}$ die ionischen Aktivitäten von $NO_3^-$, $H_2PO_4$; $SO_4^{2-}$, $K^+$, $Ca^{2+}$, $Mg^{2+}$, $NH_4^+$, $Fe^{2+}$ in den Nährlösungen darstellen.

3. Verfahren nach Anspruch 1 oder 2, **dadurch gekennzeichnet, dass** die multivariaten linearen Regressionsgleichungen des EC der Nährlösung unter Verwendung der mittleren Ionenaktivitäten *T* der Hauptzusammensetzungen der Nährlösung mit der spezifischen Formel geschätzt werden; und die multivariaten linearen Regressionsmodelle auf der Grundlage der mittleren Ionenaktivitäten der Zusammensetzungen in der experimentellen Gartenbau-Nährlösung und der Yamasaki-Tomaten-Nährlösung und ihres EC wie folgt sind:

| Formel | EC/pH | Multivariate lineare Regressionsgleichungen | $R^2$ |
|---|---|---|---|
| Experimentelle Nährstoffformel für den Gartenbau | EC | EC = $0.020+0.286T_{KNO3}-0.098T_{Ca(NO3)2\cdot4H2O}+0.671T_{Mgso4}-0.21\ 3T_{NH4H2PO4}$ | 0,9997 |
| Yamasaki Tomaten-Nährstoffformel | EC | EC = $0.013-0.2701_{KNO3}+0.846T_{Ca(No3)2\cdot4H2O}+0.256T_{Mgso4}+0.106\ T_{NH4H2PO4}$ | 0,9997 |

**4.** Verfahren nach Anspruch 1 oder 2, **dadurch gekennzeichnet, dass** zur Abschätzung des EC die linearen Regressionsmodelle der mittleren ionischen Aktivität *T* und EC in der Nährlösung mit der spezifischen Formel nach Zugabe eines bestimmten Einzelsalzes verwendet werden; die multivariaten linearen Regressionsmodelle, die auf der mittleren Ionenaktivität des spezifischen zugesetzten Einzelsalzes und deren EC in der gartenbaulichen experimentellen Nährlösung (HENS) und der Yamasaki-Tomatennährlösung (YTNS) basieren, lauten wie folgt:

| Hinzugegebene einzelne Salze | HENS Lineare Regressionsgleichungen | $R^2$ | YTNS Lineare Regressionsgleichungen | $R^2$ |
|---|---|---|---|---|
| $K_2SO_4$ | $EC=0{,}156T_i+2{,}321$ | 0,9996 | $EC=0{,}168T_i+1{,}154$ | 0,9992 |
| $KNO_3$ | $EC=0{,}145T_i+2{,}317$ | 0,9989 | $EC=0{,}148T_i+1{,}157$ | 0,9995 |
| $KCl$ | $EC=0{,}156T_i+2{,}320$ | 0,9994 | $EC=0{,}151T_i+1{,}164$ | 0,9992 |
| $KH_2PO_4$ | $EC=0{,}101T_i+2{,}334$ | 0,999 | $EC=0{,}106T_i+1{,}163$ | 0,9978 |
| $Ca(NO_3)_2 \cdot 4H_2O$ | $EC=0{,}145T_i+2{,}318$ | 0,9972 | $EC=0{,}147T_i+1{,}159$ | 0,9994 |
| $MgSO_4 \cdot 7H_2O$ | $EC=0{,}159T_i+2{,}329$ | 0,9977 | $EC=0{,}177T_i+1{,}166$ | 0,9995 |
| $NH_4H_2PO_4$ | $EC=0{,}102T_i+2{,}332$ | 0,9957 | $EC=0{,}104T_i+1{,}163$ | 0,9986 |

**5.** Verfahren nach Anspruch 1 oder 2, **dadurch gekennzeichnet, dass** die multivariaten linearen Regressionsmodelle auf der Grundlage der mittleren ionischen Aktivität T des spezifischen zugesetzten Einzelsalzes und der mittleren ionischen Aktivität T der anderen Einzelsalze in der Nährlösung mit der spezifischen Formel und deren EC erstellt werden; und die multivariaten linearen Regressionsmodelle, die auf der mittleren Ionenaktivität des spezifischen zugesetzten Einzelsalzes und der mittleren Ionenaktivität anderer Zusammensetzungen in der experimentellen Nährlösung für den Gartenbau (HENS) und der Yamasaki-Tomatennährlösung (YTNS) basieren und ihre EC lauten wie folgt:

| Formel | zugegebene einzelne Salze | Multivariate lineare Regressionsmodelle | $R^2$ |
|---|---|---|---|
| HENS | $K_2SO_4$ | $EC=340.9-0.0863T_x-111.8T_{Ca(NO3)2}-0.0807T_{KNO3}-0.8521T_{MgSO4}+97.49T_{NH4H2PO4}$ | 0,9996 |
| | $KNO_3$ | $EC=1508+0.0847T_x-0.2729T_{Ca(NO3)2}-0.1808T_{KNO3}-1451T_{MgSO4}+369.4T_{NH4H2PO4}$ | 0,9996 |
| | $KCl$ | $EC=-7731+2.5254T_x+1656T_{Ca(NO3)2}+2.2094T_{KNO3}+2550\ T_{MgSO4}-2060_{TNH4H2PO4}$ | 0,9996 |
| | $KH_2PO_4$ | $EC=-386.6-0.6181T_i-704T_{Ca(NO3)2}-0.8554T_{KNO3}+2419\ T_{MgSO4}-0.2049T_{NH4H2PO4}$ i | 0,9991 |
| | $Ca(NO_3)_2$ | $EC=1422-0.0119T_x-0.5781T_{Ca(NO3)2}+0.2657T_{KNO3}-1358T_{MgSO4}+341.5T_{NH4H2PO4}$ | 0,9999 |
| | $MgSO_4$ | $EC=-9064+0.0094.T_x+8507T_{Ca(NO3)2}-4.562T_{KNO3}-0.263\ T_{MgSO4}-2130T_{NH4H2PO4}$ | 0,9972 |
| | $NH_4H_2PO_4$ | $EC=478.1+1.0418T_x-1841T_{Ca(NO3)2}+1394T_{KNO3}+0T_{MgSO4}+0.872T_{NH4H2PO4}$ | 0,9969 |

(fortgesetzt)

| Formel | zugegebene einzelne Salze | Multivariate lineare Regressionsmodelle | $R^2$ |
|---|---|---|---|
| YTNS | $K_2SO_4$ | EC=84.57+0.1481$T_x$-74.457$_{Ca(NO3)2}$-0.1214$T_{KNO3}$+ 0.262$T_{MgSO4}$+112.6$T_{NH4H2PO4}$ | 0,9998 |
| | $KNO_3$ | EC=66.59+0.1184$T_x$+0.1721$T_{Ca(NO3)2}$-0.2016$T_{KNO3}$-118$T_{MgSO4}$ +57.04$T_{NH4H2PO4}$ | 0,9999 |
| | $KCl$ | EC=959.4+0.131$T_x$-27.5$T_{Ca(NO3)2}$-0.2202$T_{KNO3}$-1824$T_{MgSO4}$+ 1036$T_{NH4H2PO4}$ | 0,9993 |
| | $KH_2PO_4$ | EC=28.84+ 0.2328$T_x$+213.27$_{Ca(NO3)2}$-0.0633$T_{KNO3}$-552.1$T_{MgSO4}$- 0.2662 $T_{NH4H2PO4}$ | 0,9998 |
| | $Ca(NO_3)_2$ | EC=494.78+0.1804$T_x$-0.0823$T_{Ca(NO3)2}$+ 0.0721$T_{KNO3}$+978.5$T_{MgSO4}$+538.3$T_{NH4H2PO4}$ | 0,9998 |
| | $MgSO_4$ | EC=-2.6905+0.0078$T_x$+ 107.3$T_{Ca(NO3)2}$-63.58$T_{KNO3}$- 0.289$T_{MgSO4}$+0.0961$T_{NH4H2PO4}$ | 0,9999 |
| | $NH_4H_2PO_4$ | EC=-121.9+0.1257$T_x$-0$T_{Ca(NO3)2}$-158$T_{KNO3}$+ 779.4$T_{MgSO4}$- 0.089$17_{NH4H2PO4}$ | 0,9991 |

Anmerkung: $T_x$ steht für die mittlere Ionenaktivität des spezifischen zugesetzten Einzelsalzes.

6. Verfahren nach Anspruch 1 oder 2, **dadurch gekennzeichnet, dass** es sich bei den zu testenden Nährlösungen um Nährlösungen handelt, die auf der Grundlage der Nährlösungsformel für Gartenbauversuche und der Yamasaki-Tomaten-Nährlösungsformel hergestellt wurden.

## Revendications

1. Un procédé de calcul d'un pourcentage de contribution à la conductivité électrique ionique (CE) et la conductivité électrique d'une solution nutritive en fonction d'une activité ionique, **caractérisé en ce que** le procédé comprend les étapes suivantes :

1) calculer le pourcentage de contribution à la CE ionique qui est proposé et utilisé pour représenter le pourcentage de contribution de chaque activité ionique à la CE dans la solution nutritive :

$$EC_i = \frac{| Z_i | T_i \lambda_\infty}{\sum(| Z_i | T_i \lambda_\infty)} \times 100\%$$

où $EC_i$ est le pourcentage de contribution à la CE ionique de l'ion i, en % ; $Z_i$ est le numéro de charge porté par l'ion i ; $T_i$ est l'activité ionique de l'ion i, en mol/L ; et $\lambda_\infty$ est la conductivité ionique équivalente limite de l'ion i, en $m^2$ S/mol ;
2) calculer l'activité ionique qui représente une concentration efficace de l'ion qui joue un rôle dans la solution électrolytique et est une concentration d'un ion qui joue un rôle électrostatique dans la solution nutritive :

$$T_i = \gamma_i C_i$$

où $T_i$ est l'activité ionique de l'ion i, en mol/L ; $\gamma_i$ est le coefficient d'activité ionique de l'ion i ; $C_i$ est la concentration ionique de l'ion i, en mol/L ;
3) calculer l'activité ionique moyenne qui représente la concentration globale ou l'activité globale des ions dans une solution saline unique ou une solution mixte :

$$T = \left(T_i^{\,v_i} \cdot T_j^{\,v_j}\right)^{\frac{1}{v_i+v_j}}$$

où $T$ est l'activité ionique moyenne d'une solution saline unique ou d'une solution mixte, en mol/L ; $T_i$ et $T_j$ sont respectivement les activités ioniques des cations et des anions constituant la solution saline unique ou la solution mixte, en mol/L ; $v_i$ et $v_j$ sont respectivement le nombre des cations et des anions constituant la solution saline unique ou la solution mixte ;

4) calculer, par l'équation limite de Debye-Hückel applicable aux caractéristiques de la solution nutritive, le coefficient d'activité ionique qui est un coefficient de correction représentant l'écart entre une solution réelle et une solution idéale :

$$-\lg\gamma_i = \frac{AZ_i^{\,2}\sqrt{I}}{1 + Br_i\sqrt{I}}$$

où $\gamma_i$ est le coefficient d'activité ionique de l'ion i ; $A$ est une constante liée à la température, 0,5091 à 25°C ; $B$ est une constante liée à la taille ionique, 0,328 à 25°C ; $Z_i$ est le numéro de charge porté par l'ion i ; $r_i$ est un paramètre de volume de l'ion i ; $I$ est la force ionique de la solution nutritive, en mol/L ;

5) calculer la force ionique qui représente l'intensité du champ électrique provoqué par les ions présents dans la solution nutritive :

$$I = \frac{1}{2}\sum C_i Z_i^{\,2}$$

où $I$ est la force ionique de la solution nutritive, en mol/L ; $C_i$ est la concentration ionique de l'ion i, en mol/L ; $Z_i$ est le numéro de charge porté par l'ion i ;

6) mesurer la CE et le pH de la solution nutritive ;

7) sur la base des activités ioniques moyennes $T$ de chaque sel unique dans la solution nutritive, estimer la CE de la solution nutritive par des modèles de régression linéaire multivariée et calculer inversement la concentration ionique en utilisant la CE réellement mesurée et le pourcentage de contribution de la CE ionique.

2. Le procédé selon la revendication 1, **caractérisé en ce que** les ions de la solution nutritive sont choisis dans le groupe constitué des principaux ions inorganiques $NO_3^-$, $H_2PO_4^-$, $SO_4^{2-}$, $NH_4^+$, $K^+$, $Ca^{2+}$, $Mg^{2+}$ et $Fe^{2+}$ qui sont étroitement liés à la croissance des plantes, les modèles de régression linéaire multivariée de la CE de la solution nutritive avec la formule spécifique sont établis sur la base de l'activité ionique des principaux ions inorganiques précédents ; et les modèles de régression linéaire multivariée de chaque activité ionique et de la CE de la solution nutritive établis sur la base d'une formule nutritive expérimentale horticole et d'une formule nutritive pour tomates de Yamasaki sont les suivants :

| Formule | EC/pH | Modèles de régression linéaire multivariée | $R^2$ |
|---|---|---|---|
| formule nutritive expérimentale horticole | EC | EC= $0.02+0.057T_1+0.642T_2+2.135T_3+0.658T_4+0.220T_5-0.329T_6-2.251T_7-0.913T_8$ | 1,0000 |
| formule nutritive pour tomates de Yamasaki | EC | EC= $0.001+0.172T_1-0.662T_2-0.926T_3-0.570T_4-0.079T_5-0.022T_6+0.738T_7+41.55T_8$ | 0,9999 |

où $T_{1-8}$ représente les activités ioniques de $NO_3^-$, $H_2PO_4^-$, $SO_4^{2-}$, $K^+$, $Ca^{2+}$, $Mg^{2+}$ $NH_4^+$, $Fe^{2+}$ dans les solutions nutritives, respectivement.

3. Le procédé selon la revendication 1 ou la revendication 2, **caractérisé en ce que** les équations de régression

EP 3 786 617 B1



linéaire multivariée de CE de la solution nutritive sont estimées en utilisant les activités ioniques moyennes $T$ des principales compositions de la solution nutritive avec la formule spécifique ; et les modèles de régression linéaire multivariée basés sur les activités ioniques moyennes des compositions dans la solution nutritive expérimentale horticole et la solution nutritive de tomates de Yamasaki et de leur CE sont les suivants :

| Formule | EC/pH | Équations de régression linéaire multivariée | $R^2$ |
|---|---|---|---|
| formule nutritive expérimentale horticole | EC | EC = 0. 020+0. 286$T_{KNO3}$-0.098$T_{Ca(NO3)2\cdot4H2O}$+0.671$T_{MgSO4}$-0.213$T_{NH4H2PO4}$ | 0.9997 |
| formule nutritive pour tomates de Yamasaki | EC | EC = 0.013-0.270$T_{KNO3}$+0.846$T_{Ca(NO3)2\cdot4H2O}$+0.256$T_{MgSO4}$+0.106$T_{NH4H2PO4}$ | 0.9997 |

4. Le procédé selon la revendication 1 ou la revendication 2, **caractérisé en ce que** les modèles de régression linéaire de l'activité ionique moyenne T et de la CE dans la solution nutritive de formule spécifique après ajout d'un sel unique spécifique sont utilisés pour estimer la CE ; les modèles de régression linéaire multivariée basés sur l'activité ionique moyenne du sel unique spécifique ajouté et leur CE dans la solution nutritive expérimentale horticole (HENS) et la solution nutritive de tomates de Yamasaki (YTNS) sont les suivants :

| Sels uniques ajoutés | HENS | | YTNS | |
|---|---|---|---|---|
| | Équations de régression linéaire | $R^2$ | Équations de régression linéaire | $R^2$ |
| $K_2SO_4$ | EC=0.156$T_i$+2.321 | 0.9996 | EC=0.168$T_i$+1.154 | 0.9992 |
| $KNO_3$ | EC=0.145$T_i$+2.317 | 0.9989 | EC=0.148$T_i$+1.157 | 0.9995 |
| KCl | EC=0.156$T_i$+2.320 | 0.9994 | EC=0.151$T_i$+1.164 | 0.9992 |
| $KH_2PO_4$ | EC=0.101$T_i$+2.334 | 0.999 | EC=0.106$T_i$+1.163 | 0.9978 |
| $Ca(NO_3)_2\cdot4H_2O$ | EC=0.145$T_i$+2.318 | 0.9972 | EC=0.147$T_i$+1.159 | 0.9994 |
| $MgSO_4\cdot7H_2O$ | EC=0.159$T_i$+2.329 | 0.9977 | EC=0.177$T_i$+1.166 | 0.9995 |
| $NH_4H_2PO_4$ | EC=0.102$T_i$+2.332 | 0.9957 | EC=0.104$T_i$+1.163 | 0.9986 |

5. Le procédé selon la revendication 1 ou la revendication 2, **caractérisé en ce que** les modèles de régression linéaire multivariée sont établis sur la base de l'activité moyenne ionique $T$ du sel unique spécifique ajouté et de l'activité ionique moyenne $T$ d'autres sels uniques dans la solution nutritive avec la formule spécifique et leur CE ; et les modèles de régression linéaire multivariée basés sur l'activité ionique moyenne du sel unique spécifique ajouté et l'activité ionique moyenne d'autres compositions dans la solution nutritive expérimentale horticole (HENS) et la solution nutritive de tomates de Yamasaki (YTNS) et leur CE sont les suivants :

| Formule | Sels uniques ajoutés | Modèles de régression linéaire multivariée | $R^2$ |
|---|---|---|---|
| HENS | $F_2SO_4$ | EC=340.9-0.0863$T_x$-111.8$T_{Ca(NO3)2}$-0.0807$T_{KNO3}$-0.8521$T_{MgSO4}$ +97. 49$T_{NH4H2PO4}$ | 0.9996 |
| | $KNO_3$ | EC=1508+0.0847$T_x$-0.2729$T_{Ca(NO3)2}$-0.1808$T_{KNO3}$-1451$T_{MgSO4}$ -I-369. 4$T_{NH4H2PO4}$ | 0.9996 |
| | KCl | EC=-7731+2.5254$T_x$+1656$T_{Ca(NO3)2}$+2.2094$T_{KNO3}$+2550$T_{MgSO4}$-2060$T_{NH4H2PO4}$ | 0.9996 |

(suite)

| Formule | Sels uniques ajoutés | Modèles de régression linéaire multivariée | $R^2$ |
|---|---|---|---|
| | $KH_2PO_4$ | $EC=-386.6-0.6181T_x-704T_{Ca(NO3)2}-0.8554T_{KNO3}+2419\ T_{MgSO4}-0.2049\ T_{NH4H2PO4}$ | 0.9991 |
| | $Ca(NO_3)_2$ | $EC=1422-0.0119T_x-0.5781T_{Ca(NO3)2}+0.2657T_{KNO3}-1358\ T_{MgSO4}+341.5\ T_{NH4H2PO4}$ | 0.9999 |
| | $MgSO_4$ | $EC=-9064+0.0094T_x+8507T_{Ca(NO3)2}-4562T_{KNO3}-0.263\ T_{MgSO4}-2130\ T_{NH4H2PO4}$ | 0.9972 |
| | $NH_4H_2PO_4$ | $EC=478.1+1.0418T_x-1841T_{Ca(NO3)2}+1394T_{KNO3}+0T_{MgSO4}+0.872T_{NH4H2PO4}$ | 0.9969 |
| | $K_2SO_4$ | $EC=84.57+0.1481T_x-74.45T_{Ca(NO3)2}-0.1214T_{KNO3}+0.262T_{MgSO4}+112.6T_{NH4H2PO4}$ | 0.9998 |
| | $KNO_3$ | $EC=66.59+0.1184T_x+0.1721T_{Ca(NO3)2}-0.2016T_{KNO3}-118T_{MgSO4}+57.04T_{NH4H2PO4}$ | 0.9999 |
| | $KCl$ | $EC=959.4+0.131T_x-27.5T_{Ca(NO3)2}-0.2202T_{KNO3}-1824T_{MgSO4}+1036T_{NH4H2PO4}$ | 0.9993 |
| YTNS | $KH_2PO_4$ | $EC=28.84+0.2328T_x+213.2T_{Ca(NO3)2}-0.0633T_{KNO3}-552.1T_{MgSO4}-0.2662\ T_{NH4H2PO4}$ | 0.9998 |
| | $Ca(NO_3)_2$ | $EC=494.78+0.1804T_x-0.0823T_{Ca(NO3)2}+0.0721T_{KMO3}-978.5T_{MgSO4}+538.3T_{NH4H2PO4}$ | 0.9998 |
| | $MgSO_4$ | $EC=-2.6905+0.0078T_x+107.3T_{Ca(NO3)2}-63.58T_{KNO3}-0.289T_{MgSO4}+0.0961T_{NH4H2PO4}$ | 0.9999 |
| | $NH_4H_2PO_4$ | $EC=-121.9+0.1257T_x+0T_{Ca(NO3)2}-158T_{KNO3}+779.4T_{MgSO4}-0.0891T_{NH4H2PO4}$ | 0.9991 |

Remarque : $T_x$ représente l'activité ionique moyenne du sel unique spécifique ajouté.

**6.** Le procédé selon la revendication 1 ou la revendication 2, **caractérisé en ce que** les solutions nutritives à tester dans le procédé sont des solutions nutritives préparées sur la base de la formule de solution nutritive expérimentale horticole et de la formule de solution nutritive de tomates de Yamasaki.

FIG. 1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MOREIRA BARRADAS et al.** A model to formulate nutritive solutions for fertigation with customized electrical conductivity and nutrient ratios. *Irrig Sci,* 2018, vol. 36, 133-142

- **MOREIRA BARRADAS et al.** A Decision Support System-Fertigation Simulator (DSS-FS) for design and optimization of sprinkler and drip irrigation systems. *Computers and Electronics in Agriculture,* 2012, vol. 86, 111-119